(19) Europäisches Patentamt
European Patent Office
Office européen des brevets

(11) Publication number : **0 482 939 A1**

# (12) EUROPEAN PATENT APPLICATION

(21) Application number : **91309863.8**

(51) Int. Cl.[5] : **C07D 401/06,** A61K 31/435

(22) Date of filing : **24.10.91**

(30) Priority : **24.10.90 JP 286460/90**

(43) Date of publication of application :
**29.04.92 Bulletin 92/18**

(84) Designated Contracting States :
**AT BE CH DE DK ES FR GB GR IT LI LU NL SE**

(71) Applicant : **ONO PHARMACEUTICAL CO., LTD.**
**1-5, Doshomachi 2-chome**
**Chuo-ku Osaka 541 (JP)**

(72) Inventor : **Okegawa, Tadao, c/o Minase Res. Inst.**
**Ono Pharm. Co. Ltd., 3-1-1 Sakurai**
**Shimamoto-cho**
**Mishima-gun, Osaka (JP)**
Inventor : **Kawamura, Masanori, c/o Minase Res. Inst.**
**Ono Pharm. Co. Ltd., 3-1-1 Sakurai**
**Shimamoto-cho**
**Mishima-gun, Osaka (JP)**

(74) Representative : **Bentham, Stephen**
**J.A. Kemp & Co. 14 South Square Gray's Inn**
**London, WC1R 5LX (GB)**

## (54) Isoquinolinone derivative.

(57) Isoquinolinone derivatives of the formula :

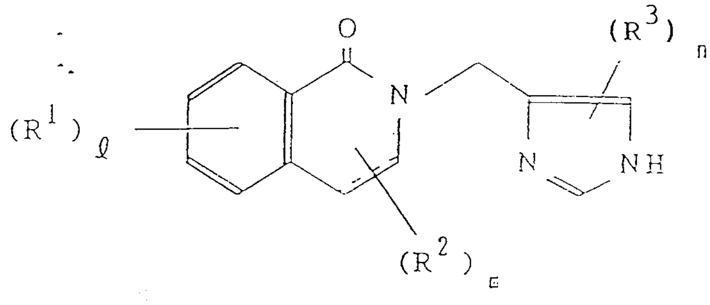

(I)

wherein $R^1$ is hydrogen, C1-4 alkyl, C1-4 alkoxy or a group of formula :

$$-NR^4R^5$$

wherein $R^4$ is hydrogen, halogen, C1-4 alkyl or C2-4 alkanoyl and
$R^5$ is hydrogen, C1-4 alkyl or benzyl ;
$R^2$ is hydrogen or C1-4 alkyl ;
$R^3$ is hydrogen or C1-4 alkyl ;
l is 1, 2, 3 or 4 ;
m is 1 or 2 ;
n is 1 or 2 and
$\overline{---}$ is a single bond or double bond and non-toxic acid addition salts thereof and hydrates thereof possess an antagonistic activity against 5-HT3 receptor, and are useful for the prevention and/or treatment of diseases induced when 5-HT acts on 5-HT3 receptors (especially vomiting induced by the administration of an anti-cancer agent).

EP 0 482 939 A1

The present invention relates to novel isoquinolinone derivatives, processes for their preparation and pharmaceutical compositions containing them.

It is well known that 5HT (hydroxytryptamine, serononin) is a neurotransmitter in the living body. Three types of receptor for the 5-HT molecule are known, 5-HT1, 5-HT2 and 5-HT3 type receptors.

5-HT3 receptors are widely distributed in the brain, heart and digestive canal, and 5-HT acts as a mediator of these receptors.

It has been confirmed that when 5-HT acts on 5-HT3 receptors at the peripheral nerve, pain and bradycardia are induced; on the other hand, when 5-HT acts on 5-HT3 receptors at the central nerve, mental actions e.g. emotions, appetite or memory are induced. Further, 5-HT acts on 5-HT3 receptors at the CTZ (chemoreceptor trigger zone) in the brain, to induce nausea and vomiting.

It is therefore thought that 5-HT3 receptor antagonists are useful in the prevention and treatment of: central nerve diseases such as schizophrenia, corpulence, mania and anxiety; gastroenteric functional defects such as peptic ulcers and peptic esophagitis; and migraine, vertigo, nausea and vomiting (especially vomiting induced by administration of an anti-cancer agent such as cisplatin).

It is known that the large scale administration of Metoclopramide suppresses vomiting induced by an anti-cancer agent but produces side effects, and that other anti-vomiting agents are not effective.

In these circumstances, development of 5-HT3 receptor antagonists has been carried out. Besides Metoclopramide mentioned above, other compounds, which are being developed have been disclosed in JP-A-60-228477 which is equivalent to EP-A-158532 (Zacopride), JP-A-58-188885, which is equivalent to EP-A-94742 (BRL 24924), JP-A-59-67284, which is equivalent to GB-A-2125398, GB-A-2166726, GB-A-2166727 and GB-A-2166278 (ICS 205,930) and JP-A-60-214784, which is equivalent to GB-A-2153821 (GR-38032F, Ondansetron).

JP-A-2-49772, which is equivalent to EP-A-336759, also discloses compounds having an antagonistic activity against 5-HT3 receptors which are of the formula (A):

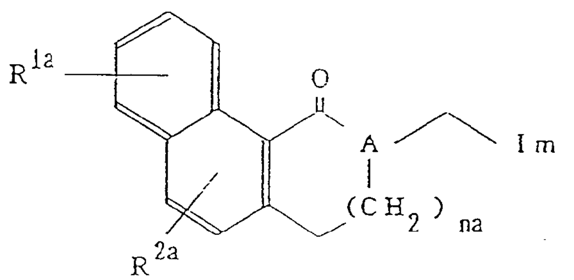

(A).

wherein Im represents an imidazolyl group of formula:

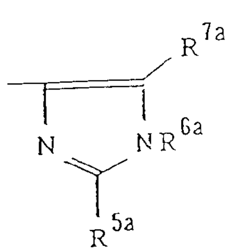

or

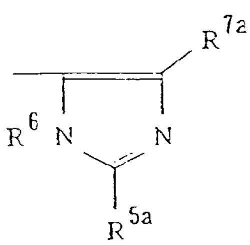

A represents the group CH or a nitrogen atom;

$R^{1a}$ and $R^{2a}$, which may be the same or different, and may be attached to either the same or different fused rings of the naphthalene moiety, each represents a hydrogen atom, a halogen atom, or a hydroxy, C1-4 alkoxy, C1-4 alkyl, or C1-4 alkylthio group, and one of $R^{1a}$ and $R^{2a}$ may also represent a group $-NR^{3a}R^{4a}$ (wherein $R^{3a}$ and $R^{4a}$, which may be the same or different, each represents a hydrogen atom or a C1-4 alkyl group, or together with the nitrogen atom to which they are attached form a saturated 5 to 7 membered ring);

na represents, 1, 2 or 3;

one of the groups represented by $R^{5a}$, $R^{6a}$ and $R^{7a}$ is a hydrogen atom or a C1-6 alkyl, C3-7 cycloalkyl, C3-6 alkenyl, phenyl or phenyl C1-3 alkyl group, and each of the other two groups, which may be the same or different, represent a hydrogen atom or a C1-6 alkyl group; and physiologically acceptable salts and solvates

thereof.

We have now devised a new series of compounds possessing antagonistic activity against 5-HT3 receptors. The compounds of the present invention have a bicyclic fused ring structure which is substituted by an imidazolylmethyl group. In contrast the compounds of the formula (A) disclosed in EP-A-336759 have a structure in which a tricyclic fused ring is substituted by imidazolylmethyl. EP-A-336759 contains no suggestion that compounds containing a bicyclic fused ring structure substituted by an imidazolylmethyl group rather than a tricyclic fused ring structure would possess 5-HT3 antagonist properties.

Accordingly, the present invention relates to an isoquinolinone derivative of the formula (I)

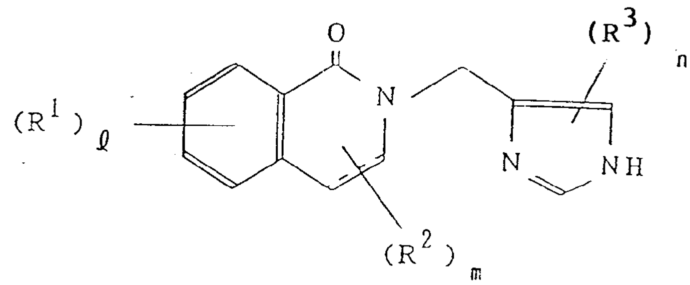

(I)

wherein each substituent $R^1$ is the same or different and is hydrogen, halogen, C1-4 alkyl, C1-4 alkoxy of a group of formula

$$-NR^4R^5$$

wherein
$R^4$ is hydrogen, C1-4 alkyl or C2-4 alkanoyl and
$R^5$ is hydrogen, C1-4 alkyl or benzyl;
each substituent $R^2$ is the same or different and is hydrogen or C1-4 alkyl;
each substituent $R^3$ is the same or different and is hydrogen or C1-4 alkyl;
l is 1, 2, 3 or 4;
m is 1 or 2;
n is 1 or 2; and
--- is a single bond or double bond;
and non-toxic acid addition salts thereof and hydrates thereof.

The invention also relates to processes for the preparation of the compounds of the invention; and pharmaceutical compositions containing them.

In the formula (I), C1-4 alkyl groups represented by $R^1$, $R^2$, $R^3$, $R^4$, or $R^5$ may be methyl, ethyl, propyl, or butyl groups.

In the formula (I), C1-4 alkoxy groups represented by $R^1$ may be methoxy, ethoxy, propoxy or butoxy groups.

In the formula (I), C2-4 alkanoyl groups represented by $R^4$ may be acetyl, propionyl or butyryl groups.

In general formula (I), halogen represented by $R^1$ means fluorine, chlorine, bromine or iodine.

Preferably in the compounds of formula (I), the imidazolyl ring is substituted by at least one methyl group, i.e, at least one of the substituents $R^3$ is methyl. More preferably the imidazolyl ring is substituted by a single methyl group (i.e. $R^3$ is methyl and n is 1), which is most preferably on the 5-position of the imidazolyl ring.

Preferably at least one of the substituents $R^2$ is hydrogen and more preferably both are hydrogen. When one of the substituents is other then hydrogen then preferably it is a methyl group.

Preferably the phenyl ring of the isoquinolinone group is substituted by at least two substituents $R^1$ (i.e. 1 is at least 2), of which one substituent is a halogen atom, preferably chlorine, and another is a group $-NR^4R^5$ as hereinbefore defined. Most preferably the phenyl ring of the isoquinoline group is substituted by halogen in the 7-position and by a group $-NR^4R^5$ in the 5- or 6-, most preferably the 6-, position; the numbering of the isoquinolinone group is shown in the Examples which follow.

Where the phenyl ring of the isoquinolinone group is substituted by an $-NR^4R^5$ group, then preferably that substituent is an amino, methylamino, acetylamino, N-acetyl N-methylamino or benzylamino substituent.

Throughout the present specification, all the groups and moieties mentioned may be present in any isomeric form unless otherwise specified. For example, alkyl or alkoxy includes straight or branched chains. The present invention includes compounds which may exist as optically active isomers generated by the existence of asymmetric carbon atoms e.g. the existence of branched alkyl. The present invention relates to such compound both

in optically pure form and also as mixtures of isomers.

The compounds of the invention may exist in more than one tautomeric form in the imidazole moiety, and the present invention includes them in either tautomeric form.

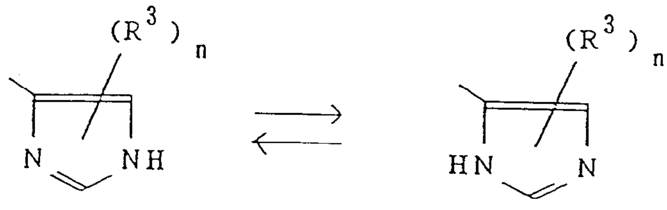

The compounds of formula (I) may be converted into the corresponding acid addition salts. Non-toxic and water soluble salts are preferable. Suitable salts include: salts of inorganic acids, such as the hydrochloride, hydrobromide, sulphate, phosphate, or nitrate; and salts of organic acids such as the acetate, lactate, tartrate, fumarate, maleate, oxalate, citrate, benzoate, methanesulphonate, ethanesulphonate, benzenesulphonate, toluenesulphonate, isethioate, glucuronaate or gluconate. The hydrochloride is preferable.

Compounds of the general formula (I) or salts thereof may be formed as hydrates or converted into hydrates by known conventional means.

Among the compounds of the formula (I), the following compounds are preferred in addition to the example compounds described hereafter:

7-chloro-6-benzylamino-2-(5-methylimidazol-4-ylmethyl)-1,2-dihydroisoquinolin-1-one,
7-chloro-6-amino-3-methyl-2-(5-methylimidazol-4-ylmethyl)-1,2-dihydroisoquinolin-1-one,
7-chloro-6-methylamino-3-methyl-2-(5-methylimidazol-4-ylmethyl)-1,2-dihydroisoquinolin-1-one,
7-chloro-6-benzylamino-3-methyl-2-(5-methylimidazol-4-ylmethyl)-1,2-dihydroisoquinolin-1-one,
7-chloro-6-amino-4-methyl-2-(5-methylimidazol-4-ylmethyl)-1,2-dihydroisoquinolin-1-one,
7-chloro-6-methylamino-4-methyl-2-(5-methylimidazol-4-ylmethyl)-1,2-dihydroisoquinolin-1-one,
7-chloro-6-benzylamino-4-methyl-2-(5-methylimidazol-4-ylmethyl)-1,2-dihydroisoquinolin-1-one,
7-chloro-6-amino-2-(5-methylimidazol-4-ylmethyl)-1,2,3,4-tetrahydroisoquinolin-1-one,
7-chloro-6-methylamino-2-(5-methylimidazol-4-ylmethyl)-1,2,3,4-tetrahydroisoquinolin-1-one,
7-chloro-6-benzylamino-2-(5-methylimidazol-4-ylmethyl)-1,2,3,4-tetrahydroisoquinolin-1-one,
7-chloro-6-amino-3-methyl-2-(5-methylimidazol-4-ylmethyl)-1,2,3,4-tetrahydroisoquinolin-1-one,
7-chloro-6-methylamino-3-methyl-2-(5-methylimidazol-4-ylmethyl)-1,2,3,4-tetrahydroisoquinolin-1-one,
7-chloro-6-benzylamino-3-methyl-2-(5-methylimidazol-4-ylmethyl)-1,2,3,4-tetrahydroisoquinolin-1-one,
7-chloro-6-amino-4-methyl-2-(5-methylimidazol-4-ylmethyl)-1,2,3,4-tetrahydroisoquinolin-1-one,
7-chloro-6-methylamino-4-methyl-2-(5-methylimidazol-4-ylmethyl)-1,2,3,4-tetrahydroisoquinolin-1-one, and
7-chloro-6-benzylamino-4-methyl-2-(5-methylimidazol-4-ylmethyl)-1,2,3,4-tetrahydroisoquinolin-1-one.

The compounds of the present invention may be prepared by hydrolysis under acidic conditions of a compounds of formula (II)

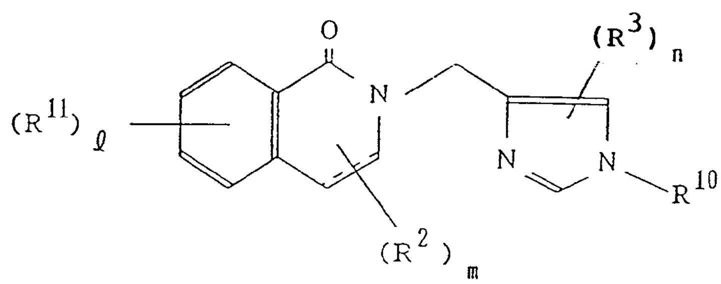

(II)

wherein $R^{10}$ is triphenylmethyl, trimethylsilyl or t-butyldimethylsilyl;
$R^{11}$ is hydrogen, halogen, C1-4 alkyl, C1-4 alkoxy or a group of formula:

$$-NR^{41}R^{5}$$

wherein $R^{41}$ is C1-4 alkyl or C2-4 alkanoyl; and the other symbols are as hereinbefore defined, and optionally converting the product thus obtained to a non-toxic acid addition salt thereof or a hydrate

thereof.

Hydrolysis under acidic conditions is a known type of reaction per se and may be carried out, for example, in a water-miscible organic solvent (for example, methanol, ethanol, THF or dioxane) an aqueous solution of organic acid (for example acetic acid, p-toluenesulphonic acid, tricloroacetic acid or oxalic acid) or using an aqueous solution of inorganic acid (for example hydrochloric acid, sulphuric acid or hydrobromic acid) or mixtures thereof, at a temperature of from 0°C - 90°C.

Compounds of formula (I) may be converted to their non-toxic acid addition salts or hydrates by well known conventional means.

The compounds of formula (II) may be prepared by following the reaction scheme (A) below, using known methodology.

In scheme (A)

$R^{20}$ and $R^{21}$ are hydrogen or C1-4 alkyl;

$R^{50}$ is C1-4 alkyl;

X is halogen and

the other symbols are as hereinbefore defined.

Scheme (A)

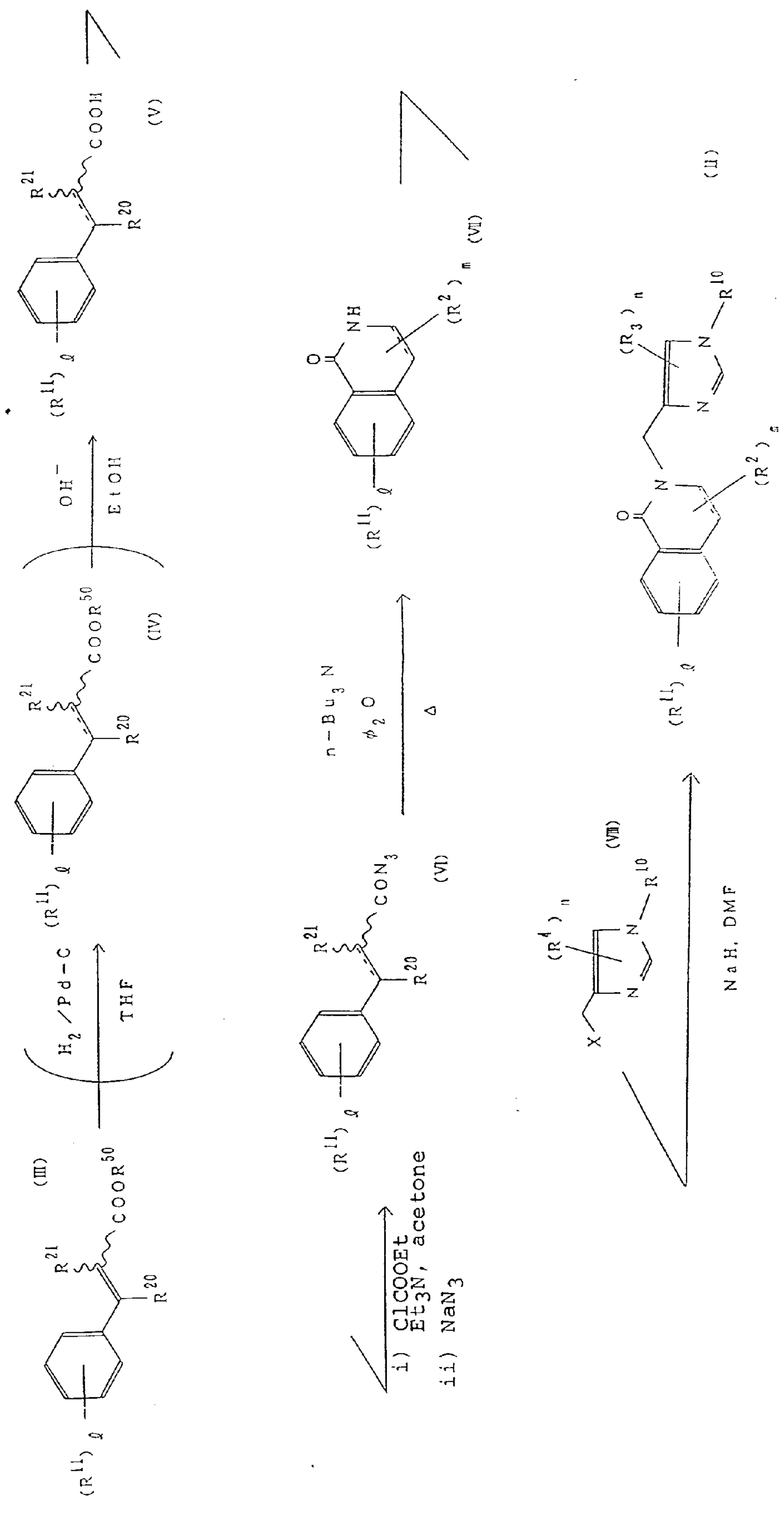
(III)
H2 /Pd-C
THF
(IV)
OH⁻
EtOH
(V)
i) ClCOOEt
Et3N, acetone
ii) NaN3
(VI)
n-Bu3N
φ2O
Δ
(VII)
(VIII)
NaH, DMF
(II)

The starting materials and reagents used in the present invention to prepare compounds of formula (I) according to Scheme (A) are known per se or may be prepared by known methods.

The compounds of the present invention possess an antagonistic activity against 5-HT3 receptors as described above, which is demonstrated by the following standard laboratory test:

Male Wister rat was urethane-anesthetised and fixed. Cannulas were inserted in the carotoid artery and in the thigh vein as used for recording of blood pressure and heart beat, and for the administration of the test compounds, respectively.

Several doses of a compound of the present invention were administered from the vein. 5-HT was administered rapidly to the vein, 2 mins. after insertion of cannulas. The suppression effect of the compounds were confirmed by the measurement of the reflex bradycardia generated (Nature 316, 126 (1985)).

Results obtained are shown in Table 1:

Table 1

An antagonistic activity against 5-HT3 receptor in vivo in rats

| Example No. of the compound | IC50 i.v. (mg/kg) |
|---|---|
| 1 | 0.39 |
| 1(a) | 0.99 |
| 1(d) | 0.49 |
| 1(e) | 1.44 |

And further, in another laboratory test, the compounds of the present invention of the formula (I) possess a potent anti-vomiting activity in vivo in ferrets.

Tests confirmed that the toxicity of the compounds of the present invention is very low. Therefore, the compounds of the present invention may be considered to be sufficiently safe and suitable for pharmaceutical use.

For example, the value of acute toxicity (LD50) of the compound prepared as in Example 1 was 50 mg/kg animal body weight by intravenous administration in mice.

It is thought that to block the activity of 5-HT3 receptor is useful for the prevention and the treatment of central nerve diseases such as schizophrenia, corpulence, mania and anxiety; gastroenteric functional defects such as peptic ulcer, and peptic esophagitis; and migraine, vertigo, nausea and vomiting (especially vomiting induced by adminstration of an anti-cancer agent such as cisplatin) in animals including human beings, but especially in human beings.

The compounds of the present invention possess an antagonistic activity against 5-HT3 receptor, shown in vivo in the experimental results above, so that they are expected to be adaptable for the uses described above.

For the purposes above described, a compound of formula (I), a non-toxic acid addition salt thereof, or a hydrate thereof, will normally be administered systemically or partially, usually by oral or parenteral administration.

The dose to be administered is determined depending upon age, body weight, symptoms, the desired therapeutic effect, the route of administration, the duration of the treatment, etc. In the human adult, the dose per person per dose is generally between 50 μg and 100 mg, preferably from 1 mg to 20 mg by oral administration, up to several times per day, and between 5 μg and 10 mg, preferably 500 μg to 10 mg by parenteral administration up to several times per day, or by continuous administration for between 1 and 24 hours per day to the vein.

As mentioned above, the doses to be used depend upon various conditions. Therefore, there are cases in which doses lower than or greater than the ranges specified above may be used.

Accordingly, the present invention also relates to a pharmaceutical composition comprising, as active ingredient, a compound of the present invention in association with a pharmaceutically acceptable carrier or diluent.

The compounds of the present invention, may be administered for example as solid compositions, liquid compositions or other compositions for oral administration or as injections, liniments or suppositories, etc. for parenteral administration.

Solid compositions for oral administration include tablets, pills, capsules, dispersible powders, granules, etc.,

Capsules include soft capsules and hard capsules.

Liquid compositions for oral administration include pharmaceutically acceptable-emulsions, solutions, suspensions, syrups and elixirs. Moreover such compositions may be employed with inert diluent(s) of the kinds commonly used (water, ethanol, etc.).

Injections for parenteral administration include sterile aqueous or non-aqueous solutions, suspensions and emulsions.

Other compositions for parenteral administration include liquids for external use, e.g. external solutions, endermic liniments, ointments, suppositories for rectal administration, pessaries, etc.

The invention will now be further illustrated by the following Examples and Reference Examples.

Examples and Reference Examples

The solvents in parentheses show the developing or eluting solvents and the ratio of the solvents used are by volume in chromatographic separations.

Unless otherwise specified, "IR" was measured by the KBr disk method.

In the following examples and reference examples, each symbols have the following meanings or the same meaning as herembefore defined.

Ac : acetyl;
Me : methyl;
Et : ethyl;
ϕ : phenyl.

Reference example 1

Synthesis of 3-acetylamino-4-chlorocinnamic acid

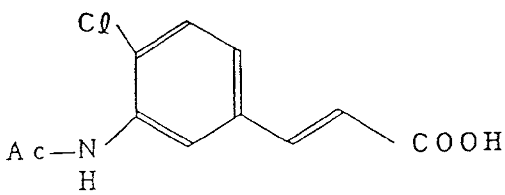

1N NaOH aq. (8.0 ml) was added to a solution of 3-amino-4-chlorocinnamic acid ethyl ester (1.95 g) in dioxane (16 ml). The solution was stirred for 15 hrs at room temperature. 1N HCl aq. (8.0 ml) was added to the solution. The solution was extracted with ethyl acetate. The extract was washed with a saturated brine, dried and evaporated. The residue was washed with a mixed solvent of hexane -EtOAc (1:3) to give the title compound (975 mg) having the following physical data:

TLC : Rf 0.10 (CHCl3: MeOH = 10: 1)

Reference example 2

Synthesis of 3-acetylamino-4-chlorocinnamoylazide

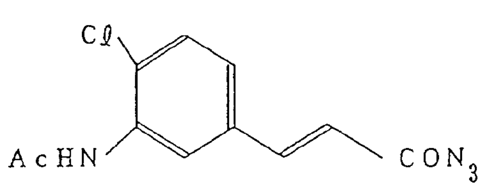

A solution of the compound prepared in reference example 1 (975 mg) and triethylamine (0.68 ml) in acetone (10 ml) was cooled with ice. Ethyl chloroformate (0.47 ml) was added dropwise to the solution. The solution was stirred for 30 mins. A solution of sodium azide (400 mg) in water (3 ml) was added to the solution dropwise. The mixture was stirred for 1 hr. After reaction, the solvent was removed by evaporation. The residue was poured into ice-water. The mixture was extracted with methylene chloride. The organic layer was washed, dried and evaporated to give the title compounds.

Reference example 3

Synthesis of 6-acetylamino-7-chloro-1,2-dihydroisoquinolin-1 -one

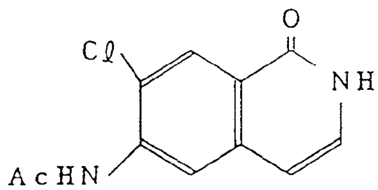

A solution of tri-n-butylamine (1.17 ml) in diphenyl ether (10 ml) was heated to 230°C. A solution of the compound prepared in reference example 2 in diphenyl ether (10 ml) was added to the solution dropwise. After cooling, hexane (20 ml) was added to the solution, deposited solids were gathered by filtration. The solids were washed and dried to give the title compound (530 mg) having the following physical data:

TLC : Rf 0.47 (CHCl3: MeOH = 10: 1)

Reference example 4

Synthesis of 6-acetylamino-7-chloro-2-(5-methyl-1-triphenylmethylimidazol-4-ylmethyl)-1,2-dihydroiso-quinolin-1-one

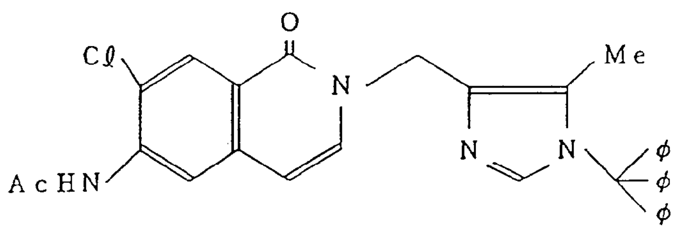

A suspension of sodium hydrate (24 mg ; 60%) in dimethylformamide (DMF) (3 ml) was cooled with ice. The compound (130 mg) prepared in reference example 3 was added to the suspension. The mixture was stirred for 10 mins at 0°C. 4-Chloromethyl-5-methyl-1-triphenylmethylimidazole (246 mg) was added to the reaction mixture. The mixture was stirred for 1 hr at room temperature and for 30 mins. at 60°C. After cooling, the reaction mixture was poured into ice-water. The mixture was extracted with EtOAc. The organic layer was washed with water and a saturated brine, dried and evaporated. The residue was purified by column chromatography on silica gel (CHCl3 : MeOH = 50 : 1) to give the title compound (230 mg) having the following physical data:

TLC : Rf 0.82 (CHCl3: MeOH = 10: 1)

Example 1

Synthesis of 6-amino-7-chloro-2-(5-methylimidazol-4-ylmethyl)-1,2-dihydroisoquinolin-1-one dihydrochloride

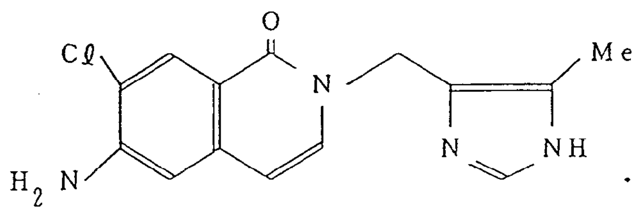

· 2HCℓ

A mixture of the compound prepared in reference example 4 (230 mg), 6N HCl (3 ml), water (3 ml), dioxane (3 ml) and methanol (1 ml) was refluxed for 30 mins. After cooling, the mixture was evaporated. The residue was purified by column chromatography on silica gel (CHCl3 : MeOH : AcOH = 30 : 5 : 1). The obtained solid (83 mg) was dissolved into methanol. A mixture of HCl and methanol was added to the solution. The solution was concentrated to dryness to give the title compound (76 mg) having the following physical data:

TLC : Rf 0.37 (CHCl3 : MeOH : AcOH = 10 : 2 : 1 )

IR : ν 3436, 1666, 1644, 1626, 1479, 1379, 908, 792 cm-1.

Example 1(a) - 1(h)

By the same method shown in Reference Example 1 to 4 and by the same procedure of Example 1, the compounds having the physical data described in following Table II were given.

The compound shown in Example 1(a), 1(b) and 1(c) were used acetic acid, instead of hydrochloric acid which was use in Example 1.

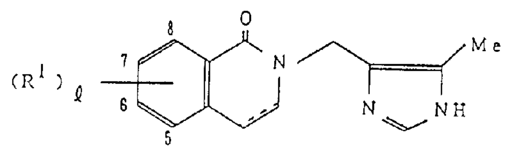

Table No. II

| Ex. No. | (R1)l-, 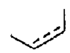 | Name | TLC(Rf) | IR (ν cm-1) |
|---|---|---|---|---|
| 1(a) | 6-acetylamino-<br>7-chloro-<br>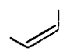 | 6-acetylamino-7-chloro-2-(5-methylimidazol-4-ylmethyl)-1,2-dihydro-isoquinolin-1-one hydrochloride | 0.38<br>($CHCl_3$:MeOH = 5:1) | 3392, 3025, 2877, 1672, 1646, 1626, 1558, 1519, 1480, 1379, 1334, 1256, 1019, 907, 792 |
| 1(b) | 6-(N-acetyl-N-methyl-amino)-<br>7-chloro- 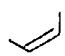 | 6-(N-acetyl-N-methylamino)-7-chloro-2-(5-methylimidazol-4-yl-methyl)-1,2-dihydro-isoquinolin-1-one hydrochloride | 0.40<br>($CHCl_3$:MeOH = 5:1) | 3319, 3086, 2603, 1651, 1622, 1959, 1455, 1396, 1365, 1285, 1201, 1119, 989, 904, 793 |
| 1(c) | 5-(N-acetyl-N-methyl-amino)-<br>7-chloro- 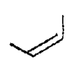 | 5-(N-acetyl-N-methylamino)-7-chloro-2-(5-methylimidazol-4-yl-methyl)-1,2-dihydro-isoquinolin-1-one hydrochloride | 0.44<br>($CHCl_3$:MeOH = 5:1) | 3278, 3096, 1651, 1624, 1593, 1549, 1483, 1422, 1392, 1373, 1330, 1287, 1156, 1014, 936, 894, 806 |

Table No. II (continuation)

| Ex. No. | $(R^1)_1$-, 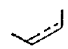 | Name | TLC(Rf) | IR (ν cm-1) |
|---|---|---|---|---|
| 1(d) | 6-methylamino-<br>7-chloro- 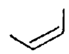 | 6-methylamino-7-chloro-2-(5-methylimidazol-4-ylmethyl)-1,2-dihydro-isoquinolin-1-one dihydrochloride | 0.40<br>($CHCl_3$:MeOH = 5:1) | 3369, 3024, 2874, 1646, 1582, 1516, 1484, 1375, 1283, 1243, 1176, 1135, 1009, 909, 841, 793 |
| 1(e) | 5-methylamino-<br>7-chloro- 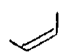 | 5-methylamino-7-chloro-2-(5-methylimidazol-4-ylmethyl)-1,2-dihydro-isoquinolin-1-one dihydrochloride | 0.32<br>($CHCl_3$:MeOH = 5:1) | 3346, 3333, 3020, 2840, 2748, 2650, 2457, 1656, 1629, 1611, 1551, 1490, 1419, 1369, 1278, 1201, 1143, 926, 802 |
| 1(f) | H 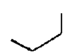 | 2-(5-methylimidazol-4-ylmethyl)-1,2,3,4-tetrahydroisoquinolin-1-one hydrochloride | 0.38<br>($CHCl_3$:MeOH = 5:1) | 3436, 3143, 2975, 2849, 2732, 2642, 1651, 1491, 1366, 1344, 1322, 1179, 921 |

Table No. II (continuation)

| Ex. No. | $(R^1)l$-, 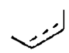 | Name | TLC(Rf) | IR (ν cm-1) |
|---|---|---|---|---|
| 1(g) | 7-methyl-<br>6-amino- 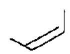 | 7-methyl-6-amino 2-(5-methylimidazol-4-ylmethyl)-1,2-dihydroisoquinolin-1-one dihydrochloride | 0.50<br>($CHCl_3$:MeOH = 3:1) | 2856, 1669, 1625, 1517, 1376, 1289, 878, 795, 691, 630 |
| 1(h) | 6-amino- 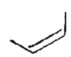 | 6-amino-2-(5-methylimidazol-4-ylmethyl)-1,2-dihydro isoquinolin-1-one dihydrochloride | 0.57<br>($CHCl_3$:MeOH:AcOH=5:5:1) | 3108, 3014, 1672, 1632, 1532, 1470, 1379, 1337, 1291, 1243, 1177, 1117, 928, 874, 834, 787, 684, 630 |

Formulation Example 1

The following components were admixed by conventional methods and punched out to obtain 100 tablets each containing 20 mg of active ingredient.

- 6-amino-7-chloro-2-(5-methylimidazol-4-ylmethyl)-1,2-dihydroisoquinolin-1-one dihydrochloride --- 1.0g
- Calcium cellulose glycolate (carboxymethylcellulose calcium; disintegrating agent) --- 0.2 g
- Magnesium stearate (lubricating agent) --- 0.1 g
- Lactose --- 8.7 g

Formulation Example 2

The following components were admixed in conventional manner. The solution was sterilized in conventional manner, placed in 1 ml portions into 5 ml ampoules and freeze-dried to obtain 100 ampoules each containing 2 mg of the active ingredient.

- 6-amino-7-chloro-2-(5-methylimidazol-4-ylmethyl)-1,2-dihydroisquinolin-1-one dihydrochloride --- 0.2 g
- Lactose --- 2 g
- Distilled water --- 100 ml

**Claims**

1. An isoquinolinone derivative of formula (I):

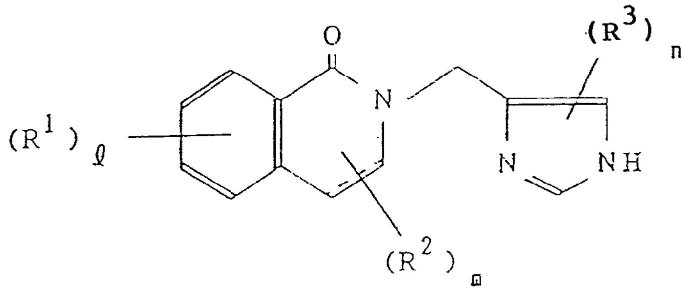

(I)

wherein each substituent $R^1$ is the same or different and is hydrogen, halogen, C1-4 alkyl, C1-4 alkoxy or a group of formula:

$$-NR^4R^5$$

wherein $R^4$ is hydrogen, C1-4 alkyl or C2-4 alkanoyl and
$R^5$ is hydrogen, C1-4 alkyl or benzyl;
each substituent $R^2$ is the same or different and is hydrogen or C1-4 alkyl;
each substituent $R^3$ is the same or different and is hydrogen or C1-4 alkyl;
l is 1, 2, 3 or 4;
m is 1 or 2;
n is 1 or 2 and
--- is a single bond or double bond; or a non-toxic acid addition salt thereof or a hydrate thereof.

2. A compound according to claim 1, wherein at least one of the substituents $R^3$ is methyl.

3. A compound according to claim 1 or 2 wherein at least one of the substituents $R^2$ is hydrogen.

4. A compound according to claim 3, which is:
2-(5-methylimidazol-4-ylmethyl)-1,2,3,4-tetrahydroisoquinolin-1-one,
7-methyl-6-amino-2-(5-methylimidazol-4-ylmethyl)-1,2-dihydroisoquinolin-1-one or
6-amino-2-(5-methylimidazol-4-ylmethyl)-1,2-dihydroisoquinolin-1-one;
or a non-toxic acid addition salt thereof or a hydrate thereof.

5. A compound according to claim 1, 2 or 3 wherein 1 is at least 2 and at least one of the substituents $R^1$ is chlorine and at least one other of the substituents $R^1$ is a group of the formula: $-NR^4R^5$ as defined in claim 1.

6. A compound according to claim 5, which is:
6-amino-7-chloro-2-(5-methylimidazol-4-ylmethyl)-1,2-dihydroisoquinolin-1-one,
6-acetylamino-7-chloro-2-(5-methylimidazol-4-ylmethyl)-1,2-dihydroisoquinolin-1-one,
6-(N-acetyl-N-methylamino)-7-chloro-2-(5-methylimidazol-4-ylmethyl)-1,2-dihydroisoquinolin-1-one,
5-(N-acetyl-N-methylamino)-7-chloro-2-(5-methylimidazol-4-ylmethyl)-1,2-dihydroisoquinolin-1-one,
6-methylamino-7-chloro-2-(5-methylimidazol-4-ylmethyl)-1,2-dihydroisoquinolin-1-one or
5-methylamino-7-chloro-2-(5-methylimidazol-4-ylmethyl)-1,2-dihydroisoquinolin-1-one;
or a non-toxic acid addition salt thereof or a hydrate thereof.

7. A process for the preparation of a compound as claimed in any one of claims 1 to 6, which comprises hydrolysing under acidic conditions a compound of formula (II):

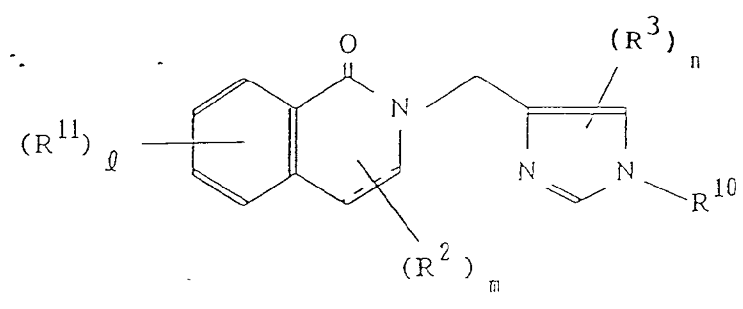

(II)

wherein $R^{10}$ is triphenylmethyl, trimethylsilyl or t-butyldimethylsilyl;
$R^{11}$ is hydrogen, halogen, C1-4 alkyl, C1-4 alkoxy or a group of the formula:

$$-NR^{41}R^5$$

wherein $R^{41}$ is C1-4 alkyl or C2-4 alkanoyl; and the other symbols are as defined in claim 1; and
optionally converting the product thus obtained to a non-toxic acid addition salt thereof, or to a hydrate thereof.

8. A pharmaceutical composition which comprises, as active ingredient, an isoquinolinone derivative of the formula (I), a non-toxic acid addition salt thereof or a hydrate thereof as claimed in any one of claims 1 to

6, in association with a pharmaceutically acceptable carrier or diluent.

**9.** A compound according to any one of claims 1 to 6 for use as a medicament.

**10.** Use of a compound as claimed in any one of claims 1 to 6 in the manufacture of a medicament for use in a method of treatment of a human or animal patient suffering from, or subject to, a condition which can be ameliorated by administration of a 5-hydroxytryptamine 3 receptor antagonist.

European Patent Office

# EUROPEAN SEARCH REPORT

Application Number

EP 91 30 9863

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 336 759 (GLAXO GROUP LIMITED) * page 4, line 15 - page 4, line 32; claims 1,9,10 * | 1,8-10 | C07D401/06 A61K31/435 |
| Y | EP-A-0 364 274 (GLAXO GROUP LIMITED) * page 4, line 17 - page 4, line 40; claims 1,9,10 * | 1,8-10 | |
| P,X | EP-A-0 405 784 (ONO PHARMACEUTICAL CO., LTD) * page 2; claims * | 1,9,10 | |
| | | | TECHNICAL FIELDS SEARCHED (Int. Cl.5) |
| | | | C07D |

The present search report has been drawn up for all claims

| Place of search | Date of completion of the search | Examiner |
|---|---|---|
| THE HAGUE | 14 JANUARY 1992 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)